# EUROPEAN PATENT APPLICATION

(11) **EP 4 174 166 A1**
(43) Date of publication of application: **03.05.2023**
(21) Application number: 20942125.4
(22) Date of filing: 26.06.2020
(51) Int. Cl.: C12N 1/20, A61K 35/74, A61P 29/00, A61P 1/16, A61P 3/00, A23L 33/135, C12R 1/01

(54) **NOVEL FAECALIBACTERIUM PRAUSNITZII STRAIN EB-FPDK9 AND USE THEREOF**

(30) Priority: 24.06.2020 KR 20200077337
(71) Applicant: ENTEROBIOME INC., Goyang-si, Gyeonggi-do 10326 (KR)
(72) Inventor: SEO, Jae Gu, Gimpo-si Gyeonggi-do 10097 (KR); SHIN, Joo Hyun, Seoul 03311 (KR); LEE, Do Kyung, Seoul 02055 (KR)
(74) Representative: Heubeck, Christian
(86) International application number: PCT/KR2020/008345
(87) International publication number: WO 2021/261631

(57) **Abstract**

The present disclosure relates to a novel *Faecalibacterium prausnitzii* EB-FPDK9 strain and the use thereof. Administration of a composition containing at least one selected from the group consisting of the *F. prausnitzii* EB-FPDK9 strain, a culture of the strain, a lysate of the strain, and an extract of the strain has the effects of preventing, ameliorating and treating inflammatory disease, liver disease or metabolic disease.

## Description

### Technical Field

The present disclosure relates to a novel *Faecalibacterium prausnitzii* strain EB-FPDK9 and the use thereof.

### Background Art

Probiotics refer to all bacteria that exhibit beneficial effects in the body, including lactic acid bacteria, and are involved in various bodily functions against bowel diseases as well as immune diseases. For a while, the study that the effect is better when dietary fiber that is the food of probiotics, that is, prebiotics, is taken together with the probiotics, has attracted attention. Recently, the assertion that postbiotics, which are metabolites released by probiotics, are effective as therapeutic agents or for diagnosis of diseases, has been attracting attention, and pharmabiotics have also been attracting attention. "Pharmabiotics" is a compound word of 'pharmaceutical' meaning medicine and 'probiotics' meaning live bacteria, refers to the human microbiome that may be used for medical purposes for disease care, and includes both probiotics and postbiotics.

Meanwhile, *Faecalibacterium* bacteria are obligate anaerobic bacilli that are always present in the intestinal mucus layer, and the retention rate and number thereof in humans are all high. In addition, these bacteria are major constituents of the intestinal flora.

Under this background, the present inventors have made efforts to develop a technology capable of curing diseases using strains harmless to the human body, and as a result, have identified a *Faecalibacterium prausnitzii* strain exhibiting an excellent anti-inflammatory effect and lipid accumulation inhibitory effect, and have found that the identified strain is suitable for the treatment of liver disease and colitis, thereby completing the present disclosure.

### DISCLOSURE

### Technical Problem

An object of the present disclosure is to provide a *Faecalibacterium prausnitzii* EB-FPDK9 strain (accession number: KCCM12620P).

Another object of the present disclosure is to provide a pharmaceutical composition for preventing or treating inflammatory disease, liver disease or metabolic disease, the pharmaceutical composition containing at least one selected from the group consisting of the *F. prausnitzii* EB-FPDK9 strain, a culture of the *F. prausnitzii* EB-FPDK9 strain, a lysate of the strain, and an extract of the strain.

Still another object of the present disclosure is to provide a food composition for preventing or ameliorating inflammatory disease, liver disease or metabolic disease, the food composition containing at least one selected from the group consisting of the *F. prausnitzii* EB-FPDK9 strain, a culture of the *F. prausnitzii* EB-FPDK9 strain, a lysate of the strain, and an extract of the strain.

### Technical Solution

One aspect of the present disclosure provides a *Faecalibacterium prausnitzii* EB-FPDK9 strain (accession number: KCCM12620P).

In one embodiment of the present disclosure, the *Faecalibacterium prausnitzii* EB-FPDK9 strain has the 16S rRNA sequence of SEQ ID NO: 1.

Another aspect of the present disclosure provides a pharmaceutical composition for preventing or treating inflammatory disease, the pharmaceutical composition containing at least one selected from the group consisting of the *F. prausnitzii* EB-FPDK9 strain, a culture of the *F*. *prausnitzii* EB-FPDK9 strain, a lysate of the strain, and an extract of the strain.

Still another aspect of the present disclosure provides a pharmaceutical composition for preventing or treating liver disease, the pharmaceutical composition containing at least one selected from the group consisting of the *F. prausnitzii* EB-FPDK9 strain, a culture of the *F*. *prausnitzii* EB-FPDK9 strain, a lysate of the strain, and an extract of the strain.

Yet another aspect of the present disclosure provides a pharmaceutical composition for preventing or treating metabolic disease, the pharmaceutical composition containing at least one selected from the group consisting of the *F*. *prausnitzii* EB-FPDK9 strain, a culture of the *F. prausnitzii* EB-FPDK9 strain, a lysate of the strain, and an extract of the strain.

Still yet another aspect of the present disclosure provides a food composition for preventing or ameliorating inflammatory disease, the food composition containing at least one selected from the group consisting of the *F*. *prausnitzii* EB-FPDK9 strain, a culture of the *F. prausnitzii* EB-FPDK9 strain, a lysate of the strain, and an extract of the strain.

A further aspect of the present disclosure provides a food composition for preventing or ameliorating liver disease, the food composition containing at least one selected from the group consisting of the *F. prausnitzii* EB-FPDK9 strain, a culture of the *F. prausnitzii* EB-FPDK9 strain, a lysate of the strain, and an extract of the strain.

Another further aspect of the present disclosure provides a food composition for preventing or ameliorating metabolic disease, the food composition containing at least one selected from the group consisting of the *F. prausnitzii* EB-FPDK9 strain, a culture of the *F. prausnitzii* EB-FPDK9 strain, a lysate of the strain, and an extract of the strain.

According to one embodiment of the present disclosure, the food composition may be prepared in the form of a health functional food.

According to one embodiment of the present disclosure, the food composition may be prepared in the form of a probiotic formulation.

### Advantageous Effects

Administration of a composition containing at least one selected from the group consisting of the *F. prausnitzii* EB-FPDK9 strain, a culture of the *F. prausnitzii* EB-FPDK9 strain, a lysate of the strain, and an extract of the strain has the effect of preventing, ameliorating or treating inflammatory disease, liver disease or metabolic disease.

### Brief Description of Drawings

FIG. 1 shows microscopic observation of a *F. prausnitzii* standard strain and EB-FPDK09.
FIG. 2 shows the results of electrophoresis performed after PCR of the *F. prausnitzii* standard strain and EB-FPDK9 with FP-specific primers.
FIG. 3 shows the results of electrophoresis performed after PCR of the *F. prausnitzii* standard strain and EB-FPDK9 with ERIC-1, ERIC-2 and (GTG)₅ primers.
FIG. 4 is a phylogenetic tree prepared using the 16rRNA nucleotide sequence of *F. prausnitzii* EB-FPDK9.
FIG. 5 shows the results of examining whether the *F*. *prausnitzii* standard strain and EB-FPDK9 cause hemolysis.
FIG. 6 is a graph showing the results of analyzing short-chain fatty acids in the *F. prausnitzii* standard strain and EB-FPDK9.
FIG. 7 is a graph showing the results of analyzing the mRNA expression of the inflammatory cytokine IL-8 in each of the *F. prausnitzii* standard strain and EB-FPDK9.
FIG. 8 is a graph showing the results of analyzing the concentration of the inflammatory cytokine IL-10 in each of the *F. prausnitzii* standard strain and EB-FPDK9.
FIG. 9 depicts photographs and a graph, which show the results of examining the degree of inhibition of lipid accumulation by each of the *F. prausnitzii* standard strain and EB-FPDK9.
FIG. 10 depicts photographs and a graph, which show the results of examining the degree of inhibition of lipid accumulation by a culture of each of the *F. prausnitzii* standard strain and EB-FPDK9.
FIG. 11 depicts graphs showing the results of comparing and analyzing the expression levels of genes, which are involved in adipocyte differentiation, after induction of adipogenesis upon treatment with each of the *F. prausnitzii* standard strain and EB-FPDK9.
FIG. 12 depicts graphs comparing body weight and dietary intake between *F. prausnitzii* standard strain-administered mice and EB-FPDK9 strain-administered mice in nonalcoholic steatohepatitis-induced mice.
FIG. 13 depicts graphs comparing glucose tolerance between *F. prausnitzii* standard strain-administered mice and EB-FPDK9 strain-administered mice in nonalcoholic steatohepatitis-induced mice.
FIG. 14 is a graph comparing the liver weight and shape between *F. prausnitzii* standard strain-administered mice and EB-FPDK9 strain-administered mice in nonalcoholic steatohepatitis-induced mice.
FIG. 15 is a graph comparing the spleen weight and shape between *F. prausnitzii* standard strain-administered mice and EB-FPDK9 strain-administered mice in nonalcoholic steatohepatitis-induced mice.
FIG. 16 depicts the results of analyzing and comparing blood lipid biochemical indicators of *F. prausnitzii* standard strain-administered mice and EB-FPDK9 strain-administered mice in nonalcoholic steatohepatitis-induced mice.
FIG. 17 shows the results of confirming the formation of fat droplets through H&E staining of the livers of *F. prausnitzii* standard strain-administered mice and EB-FPDK9 strain-administered mice in nonalcoholic steatohepatitis-induced mice.
FIG. 18 shows the results of comparing collagen deposition between *F. prausnitzii* standard strain-administered mice and EB-FPDK9 strain-administered mice in nonalcoholic steatohepatitis-induced mice.
FIG. 19 shows the results of comparing the degree of liver injury between *F. prausnitzii* standard strain-administered mice and EB-FPDK9 strain-administered mice in nonalcoholic steatohepatitis-induced mice by observing the expression of α-SMA in the liver.
FIG. 20 shows the results of comparing hepatic triglyceride and total cholesterol levels between *F. prausnitzii* standard strain-administered mice and EB-FPDK9 strain-administered mice in nonalcoholic steatohepatitis-induced mice.

### Best Mode

To achieve the above-described objects, one aspect of the present disclosure provides a *Faecalibacterium prausnitzii* EB-FPDK9 strain (accession number: KCCM12620P).

In one embodiment of the present disclosure, the *Faecalibacterium prausnitzii* EB-FPDK9 strain has the 16S rRNA sequence of SEQ ID NO: 1.

The *Faecalibacterium prausnitzii* is one of the most abundant bacteria among the bacteria constituting the human intestinal flora, and is a non-motile Firmicutes. The *Faecalibacterium prausnitzii* is characterized in that it is extremely sensitive to oxygen, and thus does not grow even in the presence of a very small amount of oxygen.

Another aspect of the present disclosure provides a pharmaceutical composition for preventing or treating inflammatory disease, the pharmaceutical composition containing at least one selected from the group consisting of the *F. prausnitzii* EB-FPDK9 strain, a culture of the *F*. *prausnitzii* EB-FPDK9 strain, a lysate of the strain, and an extract of the strain.

As used herein, the term "culture" may refer to a composition obtained after completion of culturing. More specifically, the culture may or may not contain cells. Thus, the culture may include a culture supernatant, a composition from which the culture supernatant has been removed, or a composition obtained by concentrating the same. The composition of the culture may further include, in addition to conventional components necessary for culturing *Faecalibacterium prausnitzii,* components that act synergistically on the growth of *Faecalibacterium prausnitzii,* and the composition including these components may be easily selected by those skilled in the art.

In addition, the strain may be in a liquid state or a dry state, and examples of drying methods for the strain include, but are not limited to, air drying, natural drying, spray drying and freeze drying.

As used herein, the term "inflammatory disease" is a generic term for diseases having inflammation as a main lesion. For example, the inflammatory disease may be any one selected from the group consisting of inflammatory skin diseases, inflammatory bowel diseases such as Crohn's disease and ulcerative colitis, hepatitis, peritonitis, osteomyelitis, cellulitis, meningitis, encephalitis, pancreatitis, cystic fibrosis, stroke, acute bronchitis, bronchitis, arthritis, articular cell arteritis, hemochromatosis, sicklemia and other hemoglobinopathies, and sepsis, and may preferably be inflammatory skin disease, colitis, chronic bronchitis, hepatitis, or osteoarthritis, but is not limited thereto.

Still another aspect of the present disclosure provides a pharmaceutical composition for preventing or treating liver disease, the pharmaceutical composition containing at least one selected from the group consisting of the *F. prausnitzii* EB-FPDK9 strain, a culture of the *F*. *prausnitzii* EB-FPDK9 strain, a lysate of the strain, and an extract of the strain.

The liver disease may be liver fibrosis or cirrhosis, acute or chronic hepatitis, fatty liver or liver cancer, and may preferably be fatty liver or hepatitis, more preferably nonalcoholic steatohepatitis, but is not limited thereto.

In the present disclosure, preventing or treating the liver disease may refer to suppressing the weight of the liver from increasing abnormally, and may refer to suppressing the length and weight of the spleen from increasing abnormally. In addition, it may refer to controlling the concentration of triglycerides, cholesterol, GOT or GPT or suppressing the concentration from increasing abnormally, and inhibiting the formation of fat droplets in liver cells, fibrosis of the liver and the expression of α-SMA. However, the preventive and therapeutic effects of the pharmaceutical composition are not limited thereto.

Yet another aspect of the present disclosure provides a pharmaceutical composition for preventing or treating metabolic disease, the pharmaceutical composition containing at least one selected from the group consisting of the *F*. *prausnitzii* EB-FPDK9 strain, a culture of the *F. prausnitzii* EB-FPDK9 strain, a lysate of the strain, and an extract of the strain.

The metabolic disease may be hyperlipidemia, diabetes, gout, dementia, obesity, hypertension, hypoglycemia, hypercholesterolemia, hemochromatosis, amyloidosis, or porphyria. The diabetes may include type 1 diabetes and type 2 diabetes. Preferably, the metabolic disease may be obesity, but is not limited thereto.

The pharmaceutical composition used in the present disclosure should be used in a pharmaceutically effective amount. As used herein, the term "pharmaceutically effective amount" refers to an amount sufficient to treat a disease at a reasonable benefit/risk ratio applicable to any medical treatment. The effective dose level of the pharmaceutical composition may be determined depending on factors including the subject's type, disease severity, age and sex, the type of infected virus, the activity of the drug, sensitivity to the drug, the time of administration, the route of administration, excretion rate, the duration of treatment, and drugs used in combination with the composition, as well as other factors well known in the medical field. The effective amount may vary depending on the route of treatment, the use of excipients, and the potential for use with other drugs, as appreciated by those skilled in the art.

The pharmaceutical composition of the present disclosure may be prepared in a pharmaceutical dosage form using a method well known in the art so as to provide rapid, sustained or delayed release of the active ingredient after administration to mammals. In the preparation of the dosage form, the active ingredient is preferably mixed or diluted with a carrier or encapsulated into a carrier in the form of a container.

Accordingly, the pharmaceutical composition of the present disclosure may be formulated for use in oral dosage forms, such as powders, granules, tablets, capsules, suspensions, emulsions, syrups or aerosols, or in the form of external preparations and patches, according to conventional methods, and may further contain a suitable carrier, excipient or diluent which is commonly used in the preparation of compositions.

Examples of a carrier, excipient and diluent that may be contained in the pharmaceutical composition of the present disclosure include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. The formulation may be prepared using diluents or excipients such as a filler, an extender, a binder, a wetting agent, a disintegrating agent and a surfactant, which are commonly used.

Still yet another aspect of the present disclosure provides a food composition for preventing or ameliorating inflammatory disease, the food composition containing at least one selected from the group consisting of the *F*. *prausnitzii* EB-FPDK9 strain, a culture of the *F. prausnitzii* EB-FPDK9 strain, a lysate of the strain, and an extract of the strain.

A further aspect of the present disclosure provides a food composition for preventing or ameliorating liver disease, the food composition containing at least one selected from the group consisting of the *F. prausnitzii* EB-FPDK9 strain, a culture of the *F. prausnitzii* EB-FPDK9 strain, a lysate of the strain, and an extract of the strain.

Another further aspect of the present disclosure provides a food composition for preventing or ameliorating metabolic disease, the food composition containing at least one selected from the group consisting of the *F. prausnitzii* EB-FPDK9 strain, a culture of the *F. prausnitzii* EB-FPDK9 strain, a lysate of the strain, and an extract of the strain.

In the present disclosure, the food composition may be used in various forms, including pills, powders, granules, needles, tablets, capsules or liquids and solutions, and the food composition of the present disclosure may be added to, for example, various foods, such as beverages, gums, teas, vitamin complexes, and health supplement foods.

There is no particular limitation on other ingredients, except that the food composition of the present disclosure contains, as an essential ingredient, the *F. prausnitzii* EB-FPDK9 strain, a culture of the *F. prausnitzii* EB-FPDK9 strain, a lysate of the strain, or an extract of the strain, or an active ingredient thereof or a physiologically acceptable salt thereof. Similar to common foods, the food composition may contain, as additional ingredients, various herbal extracts, food supplement additives or natural carbohydrates.

In addition, the food composition may further contain food supplement additives as mentioned above, and the food supplement additives may be conventional food supplement additives known in the art, and examples thereof include flavoring agents, coloring agents, fillers, and stabilizers.

Examples of the natural carbohydrates include monosaccharides such as glucose and fructose, disaccharides such as maltose and sucrose, polysaccharides such as dextrin and cyclodextrin, and sugar alcohols such as xylitol, sorbitol and erythritol. In addition to the ingredients described above, natural flavoring agents (e.g., rebaudioside A, glycyrrhizin, etc.) or synthetic flavoring agents (saccharin, aspartame, etc.) may be appropriately used as flavoring agents.

In addition to the ingredients described above, the food composition of the present disclosure may contain a variety of nutrients, vitamins, minerals (electrolytes), flavoring agents such as synthetic flavoring agents and natural flavoring agents, coloring agents and fillers (such as cheese or chocolate), pectic acid and salts thereof, alginic acid and salts thereof, organic acids, protective colloidal thickeners, pH-adjusting agents, stabilizers, preservatives, glycerin, alcohol, carbonizing agents used in carbonated beverages, and the like. In addition, the food composition may contain natural fruit juice and fruit flesh for the production of fruit juice beverages and vegetable beverages. These ingredients may be used alone or in combination.

According to one embodiment of the present disclosure, the food composition may be prepared in the form of a health functional food. As used herein, the term "health functional food" has the same meaning as "food for special health use (FoSHU)", and means a food having high pharmaceutical and medicinal effects, which is processed to efficiently exhibit bioregulatory functions in addition to nutrition supply. Here, the "functional food" means obtaining effects useful for health applications, such as nutrient control or physiological actions on the structures and functions of the human body. The food of the present disclosure may be prepared by a method commonly used in the art, and may be prepared by adding raw materials and ingredients which are commonly used in the art. In addition, any formulation of the food may also be prepared without limitation, as long as it is acceptable as food. The food composition of the present disclosure may be prepared into various types of formulations and has the advantages of being free from side effects that may occur upon long-term administration of drugs because it contains food as a raw material, unlike general drugs. In addition, owing to excellent portability thereof, the food composition of the present disclosure may be taken as a supplement for enhancing the effect of preventing or ameliorating inflammatory disease, liver disease or metabolic disease.

According to one embodiment of the present disclosure, the food composition may be prepared in the form of a probiotic formulation.

The probiotic formulation may be prepared and administered in various dosage forms according to various methods known in the art. For example, the *Faecalibacterium prausnitzii* EB-FPDK9 strain of the present disclosure, a culture thereof, or a concentrate or dried product of the culture may be prepared and administered in the form of powders, liquids and solutions, tablets, capsules, syrups, suspensions or granules by mixing with carriers which are commonly used in the pharmaceutical field. Examples of the carriers include, but are not limited thereto, binders, lubricants, disintegrants, excipients, solubilizing agents, dispersants, stabilizers, suspending agents, colors and flavorings. In addition, the administration dosage of the probiotic formulation may be appropriately selected depending on the *in vivo* absorption rate, inactivation rate and excretion rate of the active ingredient, the subject's age, sex, type, condition and disease severity, etc.

### Mode for Invention

Hereinafter, one or more embodiments will be described in more detail with reference to examples. However, these examples serve to illustrate one or more embodiments, and the scope of the present disclosure is not limited to these examples.

### Example 1: Isolation and Identification of Faecalibacterium prausnitzii EB-FPDK9 Strain

### 1.1. Acquisition and Isolation of Faecalibacterium prausnitzii Sample

To isolate *Faecalibacterium prausnitzii* from feces of a healthy Korean (female, 9 years old, BMI 15.5), according to the method of Martin, the feces were cultured using YBHI medium [brain-heart infusion medium supplemented with 0.5% w/v yeast extract (Difco), 0.1% w/v D-cellobiose and 0.1% w/v D-maltose], and then an extremely oxygen sensitive (EOS) strain was selected and isolated.

### 1.2. Microscopic Observation

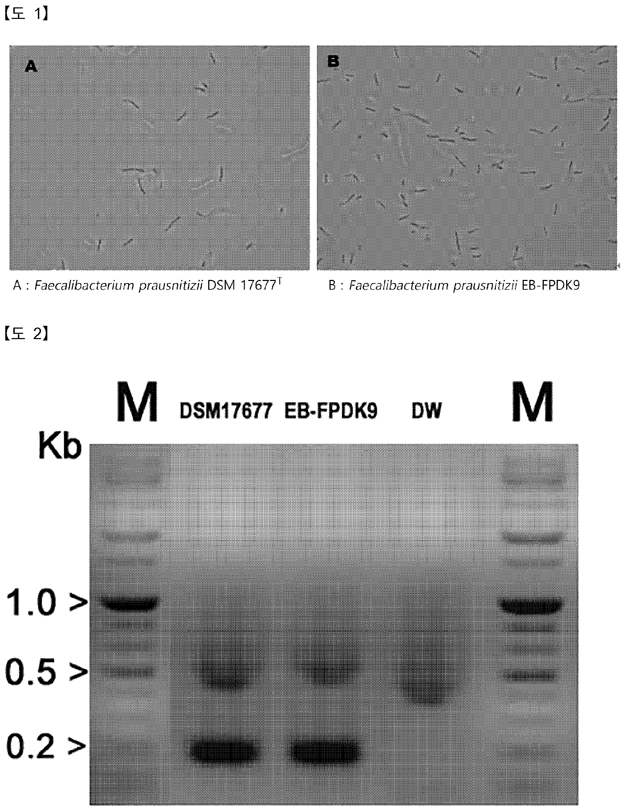

In order to confirm whether the isolated strain would be a *Faecalibacterium prausnitzii* strain, the isolated strain was observed under a microscope. As a result, as shown in FIG. 1, it was confirmed that both a *Faecalibacterium prausnitzii* DSM 17677^{T} strain as a standard strain (FIG. 1A) and the *Faecalibacterium prausnitzii* EB-FPDK9 strain observed at 1,000x magnification (FIG. 1B) had straight or curved rod cell shapes, and thus showed similar shapes.

### 1.3. PCR Analysis

In order to confirm whether the isolated strain would be a *Faecalibacterium prausnitzii* strain, the isolated strain was subjected to PCR analysis using the FP-specific primers (SEQ ID NO: 2 and SEQ ID NO: 3) shown in Table 1 below. As a result, as shown in FIG. 2, it could be confirmed that the isolated strain showed bands similar to *Faecalibacterium prausnitzii* DSM17677^{T} which is a positive control strain.

**[Table 1]**

| SEQ ID NO | Designation | Direction | Sequence (5'-3') | Amplicon size |
|---|---|---|---|---|
| SEQ ID NO: 2 | FP1 | Forward | ACT CAA CAA GGA AGT GA | 192 bp |
| SEQ ID NO: 3 | FP2 | Reverse | CAG AGG TAG GCG GAA TT | |

### 1.4. Random Amplified Polymorphic DNA (RAPD) Analysis

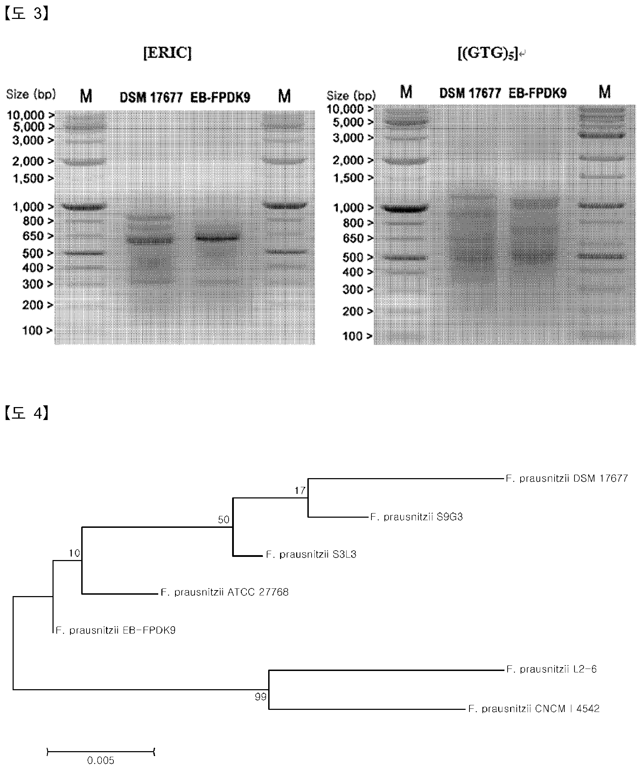

In order to check whether the strain isolated as described above is different from the previously reported standard strain of the same species, Random Amplified Polymorphic DNA (RAPD) analysis, which is a kind of molecular typing, was performed. To this end, genomic DNA (gDNA) extracted from the cells was amplified using the universal primers (SEQ ID NO: 4 to SEQ ID NO: 6) shown in Table 2 below, and then electrophoresed on 1% agarose gel for 90 minutes. Then, as shown in FIG. 3, DNA fragment patterns were compared using a UV transilluminator.

**[Table 2]**

| SEQ ID NO | Designation | Direction | Sequence (5' →3') |
|---|---|---|---|
| SEQ ID NO: 4 | ERIC-1 | Forward | ATG TAA GCT CCT GGG GAT TCA C |
| SEQ ID NO: 5 | ERIC-2 | Reverse | AAG TAA GTG ACT GGG GTG |
| | | | AGC G |
| SEQ ID NO: 6 | (GTG)₅ | Forward/Reverse | GTG GTG GTG GTG GTG |

As a result of comparing DNA fragment patterns, as shown in FIG. 3, it could be confirmed that the *Faecalibacterium prausnitzii* EB-FPDK9 strain showed band patterns, which are partially similar to but different from the standard strain *Faecalibacterium prausnitzii* DSM 17677^{T}. Thus, it was confirmed that the isolated strain is of the same species as the already reported standard strain *Faecalibacterium prausnitzii* DSM 17677^{T}, but is a strain different therefrom.

### 1.5. 16S rRNA BLAST

In order to confirm whether the isolated strain would be a *Faecalibacterium prausnitzii* strain, the isolated strain was subjected to 16S rRNA sequencing and then analyzed by BLAST. As a result, the isolated strain was 99% or more identical to *Faecalibacterium prausnitzii* species. Based on these results, the isolated strain was named the *Faecalibacterium prausnitzii* EB-FPDK9 strain, and deposited with the Korean Culture Center of Microorganisms (KCCM) under the accession number KCCM12620P.

### 1.6. Analysis of Phylogenetic Tree using 16s rRNA Nucleotide Sequence

As a result of the identification of the strain, strains similar to the currently known strains exist, but exactly consistent results were not obtained. Hence, phylogenetic tree analysis was performed. For full-length 16S rRNA gene sequencing of the isolated *Faecalibacterium prausnitzii* EB-FPDK9 strain, the 16S rRNA gene was amplified using the primers 27F (SEQ ID NO: 7) and 1492R (SEQ ID NO: 8) shown in Table 3 below, and then the nucleotide sequence thereof was determined using 3730xl DNA Analyzer (Thermo Fisher Scientific, USA). A phylogenetic tree shown in FIG. 4 was prepared according to the Maximum Likelihood method using the obtained 16S rRNA gene sequences of the EB-FPDK9 strain and the standard strain, as well as the previously published 16S rRNA gene sequences of other strains of the same species.

**[Table 3]**

| SEQ ID NO | Designation | Direction | Sequence (5' →3') | Amplicon size |
|---|---|---|---|---|
| SEQ ID NO: 7 | 27F | Forward | AGA GTT TGA TCM TGG CTC AG | 1,465 bp |
| SEQ ID NO: 8 | 1492R | Reverse | GGT TAC CTT GTT ACG ACT T | |

### Example 2: Characterization of Faecalibacterium prausnitzii EB-FPDK9 Strain

### 2.1. Examination of Antimicrobial Susceptibility

In order to examine the antimicrobial susceptibility of the *Faecalibacterium prausnitzii* EB-FPDK9 strain, the minimum inhibitory concentration (MIC) of each of antimicrobial agents piperacillin-tazobactam, ceftizoxime, chloramphenicol, clindamycin, meropenem, moxifloxacin, metronidazole, and ciprofloxacin for anaerobes against the *Faecalibacterium prausnitzii* EB-FPDK9 strain was examined according to the liquid medium microdilution method of the Clinical & Laboratory Standard Institute (CLSI) guidelines.

**[Table 4]**

| Antimicrobial agents | MIC^{a} breakpoints (µg/mL) | | | QC | Test strains | |
|---|---|---|---|---|---|---|
| | S | I | R | ATCC 29741^{b} | DSM 17677^{T} | EB-FPDK9 |
| PTZ | ≤32/4 | 64/4 | ≥128/4 | 8/4 | >256/4 (R) | 32/4 (S) |
| CTZ | ≤32 | 64 | ≥128 | 16 | 64 (I) | 128 (R) |
| CHL | ≤8 | 16 | ≥32 | 8 | 64 (R) | 32 (R) |
| CLI | ≤2 | 4 | ≥8 | 4 | ≤0.125 (S) | ≤0.125 (S) |
| MEM | ≤4 | 8 | ≥16 | 0.5 | >64 (R) | >64 (R) |
| MXF | ≤2 | 4 | ≥8 | 8 | 16 (R) | 32 (R) |
| MTZ | ≤8 | 16 | ≥32 | 2 | 4 (S) | <0.25 (S) |
| CIP | ≤1 | 2 | ≥4 | >32 | 32 (R) | >32 (R) |
| **PTZ** : Piperacillin-tazobactam, **CTZ** : ceftizoxime (3^{rd} gen), **CHL** : chloramphenicol, **CLI** : clindamycin, **MEM** : meropenem, **MXF** : moxifloxacin (4^{th} gen), **MTZ** : metronidazole, **CIP** : ciprofloxacin (2^{nd} gen), **^{a}MIC** : minimal inhibitory concentration, ^{b}*Bacteroides thetiotaomicron* ATCC 29741 | | | | | | |

As a result, as can be seen from Table 4 above, the *Faecalibacterium prausnitzii* EB-FPDK9 strain of the present disclosure showed resistance to ceftizoxime (CTZ), chloramphenicol (CHL), meropenem (MEM) and the fluoroquinolone-based antibiotics moxiproxacin (MXF) and ciprofloxacin (CIP), and showed susceptibility to piperacillin-tazobactam (PTZ), clindamycin (CLI) and metronidazole (MTZ). The *Faecalibacterium prausnitzii* EB-FPDK9 strain showed a significant difference from the standard strain (DSM 17677^{T}) with respect to the antibiotic piperacillin-tazobactam (PTZ).

### 2.2. Evaluation of Hemolytic Activity

In order to verify the safety of the *Faecalibacterium prausnitzii* EB-FPDK9 strain, evaluation was made as to whether the strain has hemolytic activity. To this end, the strain was cultured using a blood agar medium prepared by adding 1.5% w/v bacto-agar and 5% w/v defibrinated sheep blood to YBHI medium [brain-heart infusion medium supplemented with 0.5% w/v yeast extract (Difco), 0.1% w/v D-cellobiose, and 0.1% w/v D-maltose), and then observation was made as to whether hemolysis would occur around the colonies. As a positive control, *Streptococcus pyogenes* ATCC 19615 causing β-hemolysis was used for comparison.

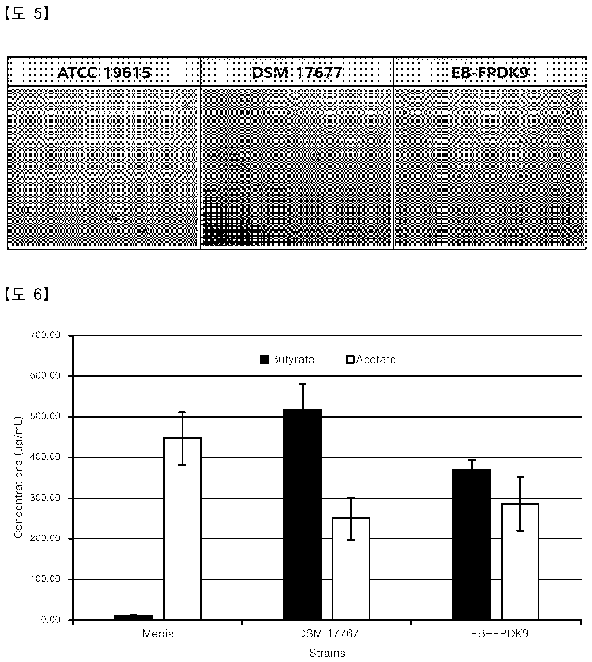

As a result, as shown in FIG. 5, both the *Faecalibacterium prausnitzii* EB-FPDK9 strain of the present disclosure and the standard strain DSM 17677^{T} showed no clear zone around the colonies, suggesting that these strains do not cause β-hemolysis associated with pathogenicity.

### 2.3. Analysis of Functional Metabolites (Short-Chain Fatty Acids)

To analyze functional metabolites in the isolated *Faecalibacterium prausnitzii* EB-FPDK9 strain, the contents of short-chain fatty acids (SCFAs) in a culture of the strain were analyzed by gas chromatography. To this end, the strain was cultured in YBHI medium [brain-heart infusion medium supplemented with 0.5% w/v yeast extract (Difco), 0.1% w/v D-cellobiose, and 0.1% w/v D-maltose) for 24 hours and then centrifuged at 12,000xg for 5 minutes. The supernatant was collected, filtered through a 0.2-um syringe filter, and then used for analysis. Analysis was performed using gas chromatography (Agilent 7890N) equipped with an FFAP column (30 m × 0.320 mm, 0.25 um phase) under the conditions shown in Table 5 below.

**[Table 5]**

| | |
|---|---|
| Flow | H₂: 40 mL/min, Air: 350 mL/min |
| Injector temp. | 240°C |
| Detector temp. | 250°C |
| Oven temp. | 40°C (hold for 2 min) → 65°C/ 10 min (hold for 2 min) →240°C/10 min (hold for 5 min) |
| Injection vol. | 2 µL |
| Split ratio | 20:1 |

As a result of analyzing the functional short-chain fatty acids, as seen from the graph in FIG. 6, it was confirmed that the *Faecalibacterium prausnitzii* EB-FPDK9 strain consumes acetate and produces butyrate.

### Example 3: Evaluation of Anti-inflammatory Effect of Faecalibacterium prausnitzii EB-FPDK9 Strain

### 3.1. Evaluation of Anti-inflammatory Effect in HT-29 Intestinal Epithelial Cells

Since cytokines are involved in the regulation of inflammatory responses in inflammatory bowel disease, the *Faecalibacterium prausnitzii* EB-FPDK9 strain was administered and changes in cytokine gene expression were examined. In order to evaluate the anti-inflammatory effect by an *in vitro* experiment, HT-29 cells (ATCC^{®} HTB-38^{™}, USA) as human colonic epithelial cells were cultured. Using, as a basal culture medium, McCoy's 5A modified medium (Gibco, USA) supplemented with 10% FBS (fetal bovine serum, Hyclone, USA) and 10 ug/ml gentamicin, the cells were cultured in an incubator (NUAIRE, USA) at 37°C under 5% CO₂. In order to confirm whether the *Faecalibacterium prausnitzii* EB-FPDK9 strain inhibits the LPS-induced expression of the inflammatory cytokine IL-8 gene in HT-29 cells, real-time PCR was performed using the primers (SEQ ID NOs: 9 to 12) shown in Table 6 below.

**[Table 6]**

| **SEQ ID NO** | **Target** | **Primer Sequence** |
|---|---|---|
| SEQ ID NO: 9 | GAPDH | F: 5'- GAC ATC AAG AAG GTG GTG AAG CAG-3' |
| SEQ ID NO: 10 | GAPDH | R: 5'- ATA CCA GGA AAT GAG CTT GAC AAA-3' |
| SEQ ID NO: 11 | IL-8 | F: 5'- TTT TGC CAA GGA GTG CTA AAG A-3' |
| SEQ ID NO: 12 | IL-8 | R: 5'- AAC CCT CTG CAC CCA GTT TTC -3' |

Total RNA was extracted using TRI reagent (Sigma, USA), and for cDNA synthesis, 1 ug of RNA was synthesized into cDNA by the M-MLV cDNA synthesis kit (Enzynomics, Korea). Real-time PCR was performed using the Quant Studio 3 real time PCR system (Applied Biosystems, USA).

Expression of the inflammatory cytokine gene was analyzed using the SYBR Green TOPrealTM qPCR 2X PreMIX (Enzynomics, Korea), and GAPDH was used as an internal standard. PCR was performed under the following conditions: pre-incubation at 50°C for 4 min and 95°C for 10 min, and 40 cycles, each consisting of 95°C for 15 sec and 60°C for 1 min. Data was analyzed by delta CT method using a program built in QuantStudio Design & Analysis Software v1.4.3.

The results obtained in all experiments were calculated as the mean and standard deviation of each experimental group using the statistical program GraphPad Prism 7 (GraphPad software Inc., USA), and the difference between groups was analyzed using one-way ANOVA, Tukey's test. A p-value ≤ 0.05 was considered significant. In some results, AUC (area under curve) was calculated.

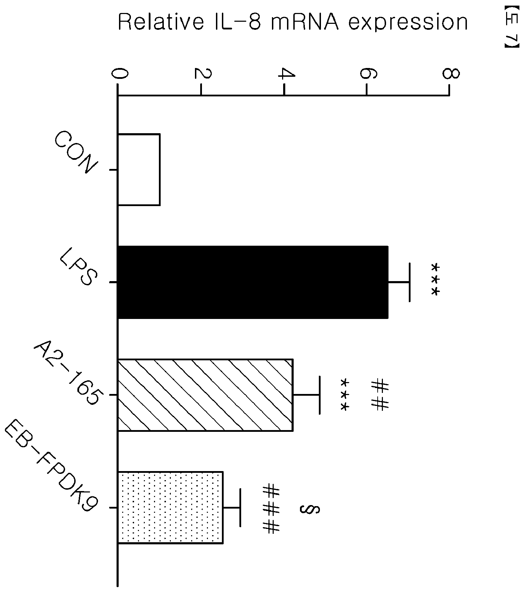

**As** a result, as shown in FIG. 7, when HT-29 cells were treated with LPS (100 ug/ml) alone for 6 hours, the expression of the representative inflammatory cytokine IL-8 in the cells significantly increased compared to that in the normal group. However, it was shown that the expression of IL-8 in the group treated with LPS together with a culture (10%, v/v) of the *Faecalibacterium prausnitzii* A2-165 standard strain decreased compared to that in the LPS-treated group, and the expression of IL-8 in the group treated with LPS together with a culture of the *Faecalibacterium prausnitzii* EB-FPDK9 strain significantly further decreased compared to that in the group treated with LPS and the A2-165 standard strain. Thus, it was confirmed that a culture of the *Faecalibacterium prausnitzii* EB-FPDK9 strain significantly decreased the inflammatory cytokine IL-8.

### 3.2. Evaluation of Anti-inflammatory Effect in Mouse Bone Marrow-Derived Dendritic Cells

To observe the anti-inflammatory response, the secretion of cytokines from dendritic cells (DC) was analyzed. To evaluate the anti-inflammatory effect of the strain using mouse bone marrow-derived dendritic cells (BMDCs), BMDCs were isolated. After a 0.5-ml microtube was punctured using an 18G needle, the femur and tibia of a 6-week-old C57BL/6 mouse were isolated and placed in a 1.5-ml precipitation tube, and then centrifuged at 10,000xg for 15 seconds. The pellet in the 1.5-ml precipitation tube was washed three times with PBS, and then the pellet was added to RPMI-1640 (10% FBS, 1% P/S, media, 1X mercaptoethanol, 20 µg GM-CSF) medium and cultured in a 150-mm culture dish. The next day, the BMDCs were transferred into and cultured in a 100-ml Petri dish, and on day 5, 10 ml of the culture was transferred into a 15-ml conical tube and then centrifuged at 1,000xg for 15 minutes. The supernatant was removed, and 10 ml of BMDC medium was added to the BMDCs and placed in a Petri dish. On day 6 or 7, the BMDCs were used in the experiment. To evaluate the anti-inflammatory effect of the *Faecalibacterium prausnitzii* EB-FPDK9 strain, the secretion of the representative anti-inflammatory cytokine IL-10 was analyzed by mIL-10 ELISA (Invitrogen, USA).

The BMDCs were treated with each of LPS (100 pg/ml), *E. coli,* the *Faecalibacterium prausnitzii* A2-165 standard strain and the EB-FPDK9 strain (10⁷ cfu/ml, 10% v/v) for 1 hour in antibiotic-free medium, and then the medium was replaced with a medium containing penicillin/streptomycin antibiotics. Next, the cells were cultured for 24 hours, and the medium was centrifuged at 1,000xg. The secretion of IL-10 was measured using the supernatant by ELISA.

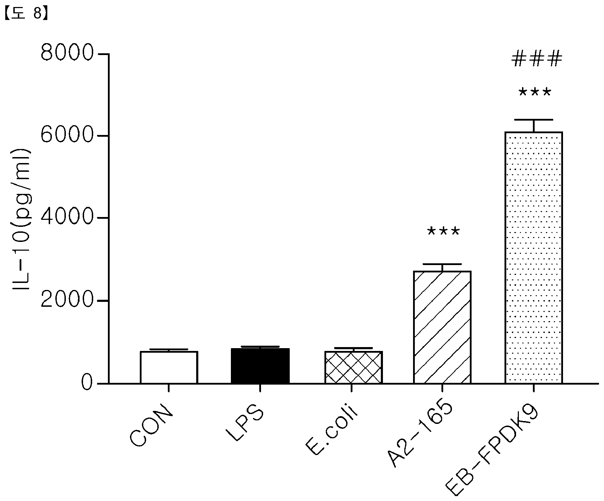

As shown in FIG. 8, the secretion of IL-10 from the cells treated with each of LPS and *E. coli* was similar to that from the normal group without a difference. However, the group treated with the *Faecalibacterium prausnitzii* A2-165 standard strain showed a significant increase in the secretion of IL-10 compared to the normal group. The expression of IL-10 in the group treated with the *Faecalibacterium prausnitzii* EB-FPDK9 strain further increased to a significant level compared to that in the group treated with the A2-165 standard strain. Thus, it was confirmed that the *Faecalibacterium prausnitzii* strain increases the anti-inflammatory cytokine IL-10 and that the *Faecalibacterium prausnitzii* EB-FPDK9 strain further increases the anti-inflammatory cytokine IL-10.

### Example 4: Evaluation of Lipid Accumulation Inhibitory Effect

Examination was made as to whether the expression of lipid accumulation- and obesity-related biomarkers is affected by administration of the strain of the present disclosure.

### 4.1. Oil Red-O Staining of Differentiated Adipocytes

In order to examine the effect of the *Faecalibacterium prausnitzii* EB-FPDK9 strain of the present disclosure on adipocyte differentiation from 3T3-L1 cells and adipogenesis, an Oil Red-O (ORO) staining experiment was performed. First, in order to allow 3T3-L1 preadipocytes to differentiate into adipocytes, cells were dispensed in a 24-well plate at a density of 2 × 10⁴/well. The cells were cultured in 10% FBS-containing DMEM medium for 4 days. When the cells reached a saturated state in the plate, the medium was replaced with differentiation medium [DMEM, 10% FBS, 0.5 mM IBMX (3-isobutyl-1-methylxanthine, Sigma I5879), 1 µM dexamethasone (Sigma D4902, FW392.5), 10 mg/ml insulin], the cells were treated with 50 µl (1 × 10⁷ cells/well) of a sample (the *Faecalibacterium prausnitzii* strain or a culture thereof) and then cultured at 37°C under 5% CO₂ for 2 days. Thereafter, the medium was replaced with insulin medium (10% FBS, 10 mg/ml insulin) every two days, and the cells were cultured under the same conditions for 8 days. The cells were treated with the *Faecalibacterium prausnitzii* strain and a culture thereof at the same time whenever the medium was replaced. The cells were treated with the strain and a culture thereof (10⁷ cfu/ml) at a concentration of 10% v/v.

Oil Red-O staining method is a method of staining differentiated 3T3-L1 cells with Oil Red-O reagent to measure fat generated in the cells. 3T3-L1 cells (Korea Cell Line Bank, Korea) as mouse preadipocytes were cultured. Using, as a basal culture medium, DMEM (Dulbecco's Modified Eagle's Medium, Welgene, Korea) supplemented with 10% FBS (fetal bovine serum, Hyclone, USA) and 1% penicillin/streptomycin, the cells were cultured in a 5% CO₂ incubator (NUAIRE, USA) at 37°C. After adipocyte differentiation from the preadipocytes 3T3-L1 was induced by insulin (1 µg/ml), IBMX (0.5 mM) and dexamethasone (1 µM) for 10 days, the culture medium was removed by washing three times with PBS, and 10% formalin (Sigma, USA) was added to the cells which were then allowed to react with an Oil Red-O (Oil red O, Sigma, USA) solution for 1 hour and washed with distilled water, thus staining fat droplets.

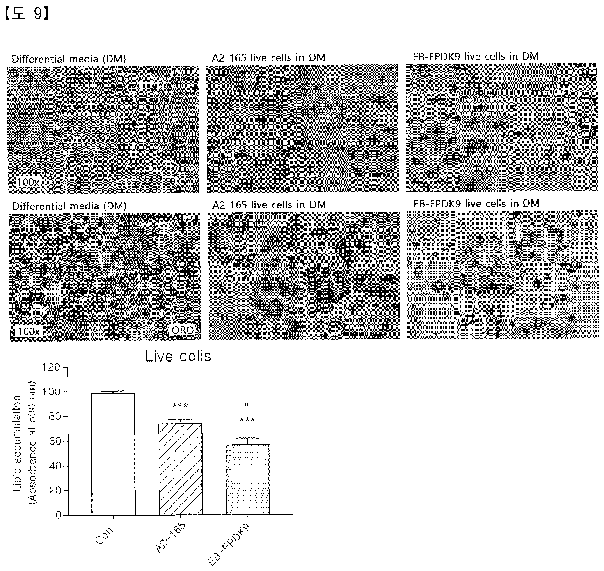

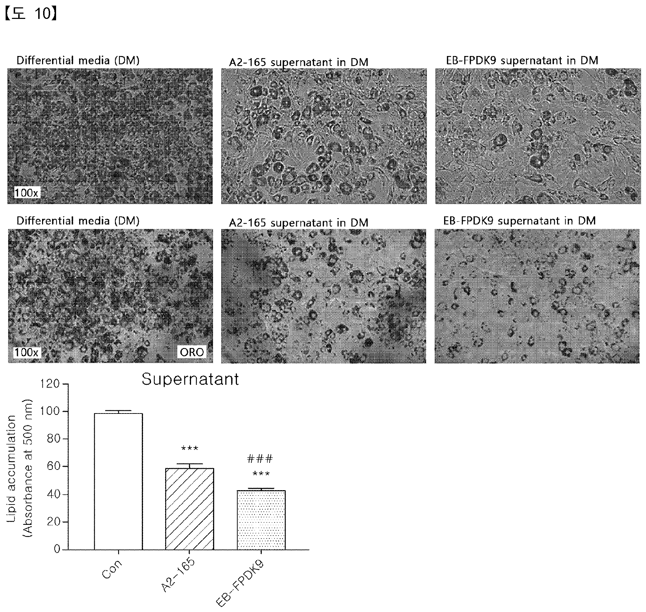

After completion of cell staining, the cells were washed three times with 40% isopropanol (Duksan, Korea) and dried, and the size of fat droplets in the cells was observed with an optical microscope. The fat droplet sample stained with the Oil Red-O solution was melted by adding isopropanol thereto, and the absorbance at 500 nm was measured using a spectrophotometer (Epoch, BioTek, USA), and the results are shown in FIGS. 9 and 10.

As shown in FIG. 9, as a result of treating 3T3-L1 cells with the *Faecalibacterium prausnitzii* A2-165 standard strain of the present disclosure during differentiation of the cells, lipid accumulation in the treated cells was inhibited compared to that in the control group. It was confirmed that treatment with the *Faecalibacterium prausnitzii* EB-FPDK9 strain more significantly inhibited lipid accumulation compared to that in the group treated with the *Faecalibacterium prausnitzii* A2-165 standard strain.

Similarly, as shown in FIG. 10, as a result of treating 3T3-L1 cells with a culture of the *Faecalibacterium prausnitzii* A2-165 standard strain during differentiation of the cells, lipid accumulation in the treated cells was significantly inhibited compared to that in the control group. Treatment with a culture of the *Faecalibacterium prausnitzii* EB-FPDK9 strain more significantly inhibited lipid accumulation compared to that in the group treated with a culture of the *Faecalibacterium prausnitzii* A2-165 standard strain.

It was confirmed that the *Faecalibacterium prausnitzii* EB-FPDK9 strain and a culture thereof have a better effect on the inhibition of adipogenic differentiation of 3T3-L1 cells than the *Faecalibacterium prausnitzii* A2-165 standard strain and a culture thereof.

### 4.2. Evaluation of Effect against Biomarker Gene Expression

In order to evaluate the effect of the strain on the inhibition of adipocyte differentiation, the mRNA expression levels of the transcription factors C/EBPα (CCAAT/enhancer binding protein alpha) and SREBP1c (sterol regulatory element-binding protein 1c) and the lipogenesis genes aP2 (adipocyte protein 2), FAS (fatty acid synthase), ACC1 (acetyl-coenzyme A-carboxylase) and LPL (lipoprotein lipase), which are involved in adipocyte differentiation and maturation at the stage of adipocyte differentiation, were analyzed by performing real-time PCR using the gene-specific primers (SEQ ID NOs: 13 to 26) shown in Table 7 below.

**[Table 7]**

| SEQ ID NO | **Target** | **Primer sequence** |
|---|---|---|
| SEQ ID NO: 13 | GAPDH | F: 5'-GAC ATC AAG AAG GTG GTG AAG CAG-3' |
| SEQ ID NO: 14 | GAPDH | R: 5'-ATA CCA GGA AAT GAG CTT GAC AAA-3' |
| SEQ ID NO: 15 | C/EBPα | F: 5'-AGC AAC GAG TAC CGG GTA CG-3' |
| SEQ ID NO: 16 | C/EBPα | R: 5'-TGT TTG GCT TTA TCT CGG CTC-3' |
| SEQ ID NO: 17 | SREBP1c | F: 5'-GAT GTG CGA ACT GGA CA -3' |
| SEQ ID NO: 18 | SREBP1c | R: 5'-CAT AGG GGG CGT CAA AGA G -3' |
| SEQ ID NO: 19 | aP2 | F: 5'-AGT GAA AAC TTC GAT GAT TAC ATG AA-3' |
| SEQ ID NO: 20 | aP2 | R: 5'-GCC TGC CAC TTT CCT TGT G-3' |
| SEQ ID NO: 21 | FAS | F: 5'-AGG GGT CGA CCT GGT CCT CA-3' |
| SEQ ID NO: 22 | FAS | R: 5'-GCC ATG CCC AGA GGG TGG TT-3' |
| SEQ ID NO: 23 | ACC1 | F: 5'-CCT CCG TCA GCT CAG ATA CA-3' |
| SEQ ID NO: 24 | ACC1 | R: 5'-TTT ACT AGG TGC AAG CCA GAC A-3' |
| SEQ ID NO: 25 | LPL | F: 5'-TTG CCC TAA GGA CCC CTG AA-3' |
| SEQ ID NO: 26 | LPL | R: 5'-ACA GAG TCT GCT AAT CCA GGA AT-3' |

Specifically, total RNA was extracted from the cell monolayer using TRI reagent (Sigma, USA) according to the manufacturer's instructions, and cDNA was synthesized from 1 µg of total RNA using the M-MLV cDNA synthesis kit (Enzynomics, Korea). A PCR reaction was performed using the Quant Studio 3 real time PCR system (Applied Biosystems, USA). The PCR was performed under the following conditions: pre-incubation at 50°C for 4 min and 95°C for 10 min, and 40 cycles, each consisting of 95°C for 15 sec and 60°C for 1 min. Data was analyzed by delta CT method using a program built in QuantStudio Design & Analysis Software v1.4.3.

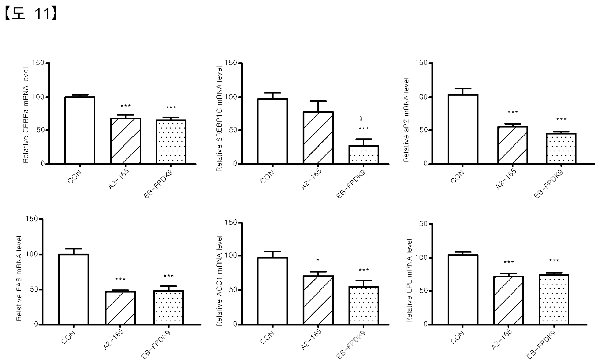

As shown in FIG. 11, when the increased expression levels of C/EBPa, SREBP1c, aP2, FAS, ACC1 and LPL, which are genes involved in adipocyte differentiation, after induction of adipogenic differentiation, were expressed as 100%, the expression levels of C/EBPa, aP2, FAS, ACC1 and LPL in the groups treated with a culture of the *Faecalibacterium prausnitzii* A2-165 standard strain decreased, and the expression levels of C/EBPa, SREBP1c, aP2, FAS, ACC1 and LPL in the group treated with a culture of the *Faecalibacterium prausnitzii* EB-FPDK9 strain significantly decreased. The expression level of SREBP1c decreased only in the group treated with a culture of the *Faecalibacterium prausnitzii* EB-FPDK9 strain compared to the control group, and the *Faecalibacterium prausnitzii* EB-FPDK9 strain further decreased the expression of all the above genes compared to the *Faecalibacterium prausnitzii* A2-165 standard strain. It was confirmed that both the *Faecalibacterium prausnitzii* A2-165 standard strain and the *Faecalibacterium prausnitzii* EB-FPDK9 strain have an effect of inhibiting the expression of adipogenic differentiation-related genes in 3T3-L1 cells.

### Example 5: Evaluation of Effect against Nonalcoholic Steatohepatitis

### 5.1. Construction of Nonalcoholic Steatohepatitis Animal Model

Animal experiments were conducted in compliance with the Animal Use and Care Protocol of the Institutional Animal Care and Use Committee (IACUC). As experimental animals, 8-week-old male C57BL/6 mice (9 mice per group) were purchased and acclimated for 1 week. Then, the mice were bred for 12 weeks. Regarding the breeding environment, the mice were acclimated for 1 week at a constant temperature (22°C) and relative humidity (40 to 60%) with a 12-hr light/12-hr dark cycle.

In order to induce nonalcoholic steatohepatitis, the mice were allowed to consume drinking water containing high-fat feed (60 kcal% fat; Research Diets Inc., NJ, USA) as an experimental diet (NASH) and 30% fructose for 16 weeks, and were allowed to access drinking water *ad libitum.*

The experimental mice were randomly divided into 5 groups as shown in Table 8 below.

**[Table 8]**

| Experimental group I (normal) | Normal diet normal control group |
|---|---|
| Experimental group II (HFD) | Group in which nonalcoholic steatohepatitis was induced by feeding experimental diet |
| Experimental group III (silymarin) | Group to which silymarin (30 mg/kg) was administered after induction of nonalcoholic steatohepatitis by feeding experimental diet |
| Experimental group IV | Group to which *Faecalibacterium prausnitzii* A2-165 standard strain was administered after induction of nonalcoholic steatohepatitis by feeding experimental diet |
| Experimental group V | Group to which *Faecalibacterium prausnitzii* EB-FPDK9 strain was administered after induction of nonalcoholic steatohepatitis by feeding experimental diet |

In the case of experimental groups III, IV and V, silymarin (30 mg/kg) or *Faecalibacterium prausnitzii* live cells at a concentration of 1×10⁸ CFU/150 µl PBS (25% glycerol and 0.05% cysteine/PBS) were orally administered daily from 8 weeks after the induction of nonalcoholic steatohepatitis by the experimental diet.

The mice of the normal diet group (Normal) were allowed to consume 10% fat feed. As a positive control, silymarin known as a functional raw material that can help ameliorate nonalcoholic fatty liver, or the *Faecalibacterium prausnitzii* A2-165 standard strain, was administered. At this time, the normal diet group and the experimental diet groups were orally administered the same amount of phosphate buffered saline (25% glycerol and 0.05% cysteine/PBS) daily in order to exclude the effect of stress or the like caused by administration.

### 5.2. Changes in Body Weight and Food Intake

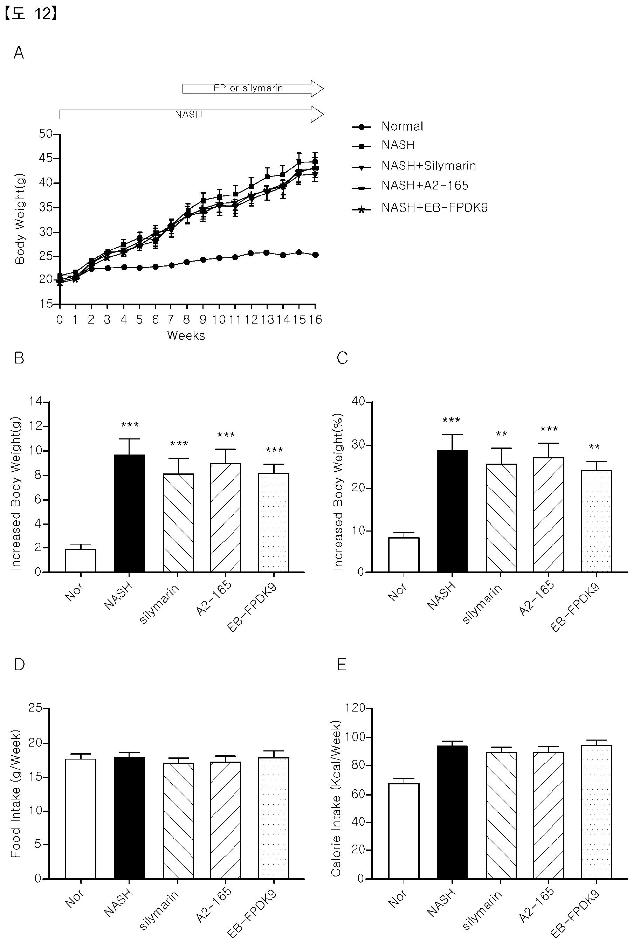

16 Weeks after performing the nonalcoholic steatohepatitis induction experiment, changes in the body weights of the experimental groups were measured, and the results are shown in FIG. 12.

Referring to FIG. 12, the body weights of all the group animals with nonalcoholic steatohepatitis induced by the experimental diet increased compared to that of the normal diet group. When the weight gain during a period from week 8 (when silymarin or the *Faecalibacterium prausnitzii* strain was administered) to week 16 was calculated as mass (g) and percentage (%), it was observed that the weight gain slightly decreased in the silymarin-administered group and the *Faecalibacterium prausnitzii* EB-FPDK9 strain-administered group compared to the nonalcoholic steatohepatitis-induced group, but a significant decrease in the weight gain could not be found. The percent weight gain was observed to be the smallest in the *Faecalibacterium prausnitzii* EB-FPDK9 strain-administered group compared to that in the normal diet group. Food intake and calorie intake did not significantly differ between the groups with nonalcoholic steatohepatitis induced by the experimental diet.

### 5.3. Changes in Glucose Tolerance (Oral Glucose Tolerance Test (OGTT))

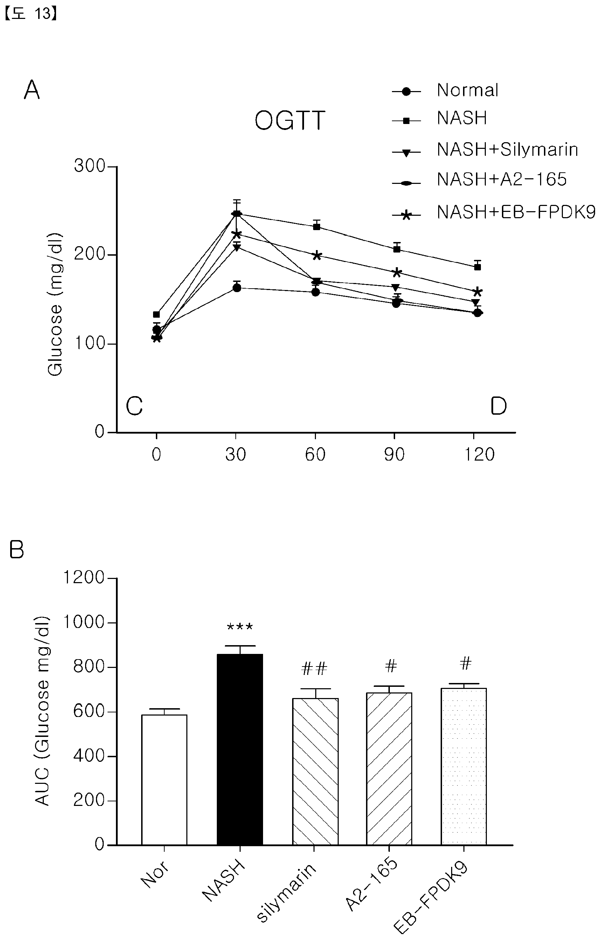

To evaluate the effect of administration of the *Faecalibacterium prausnitzii* EB-FPDK9 strain on glucose tolerance, 16 weeks after the start of the experiment, glucose (2 g/kg) were orally administered to the mice in a state in which the mice were fasted for 18 hours. Immediately before glucose administration and 30, 60, 90 and 120 minutes after glucose administration, blood was collected from the tail vein and blood glucose levels were measured with a glucometer. The results of the measurement are shown in FIG. 13.

Referring to FIG. 13, the group to which the *Faecalibacterium prausnitzii* EB-FPDK9 strain was administered immediately before glucose administration showed the greatest decrease in the blood glucose level among the administered groups. 30 minutes after glucose administration, the blood glucose level increased in all the administered groups compared to the normal diet group, but as a result of calculating the area under the curve (AUC) of the blood glucose level for 120 minutes, the blood glucose level significantly decreased in the silymarin-administered group, the *Faecalibacterium prausnitzii* A2-165 standard strain-administered group and the EB-FPDK9 strain-administered group compared to the nonalcoholic steatohepatitis-induced group as the time increased to 60 minutes, 90 minutes and 120 minutes. As a result of this study, it was confirmed that oral administration of the *Faecalibacterium prausnitzii* EB-FPDK9 strain can improve the blood glucose control ability lowered by induction of nonalcoholic steatohepatitis, and can increase glucose tolerance.

### 5.4. Observation of Steatohepatitis and Changes in Tissue Weight

At the end of the experiment, the liver and spleen were extracted under anesthesia with CO₂, washed with physiological saline, and dewatered, and then weighed, and the sizes and colors thereof were visually observed.

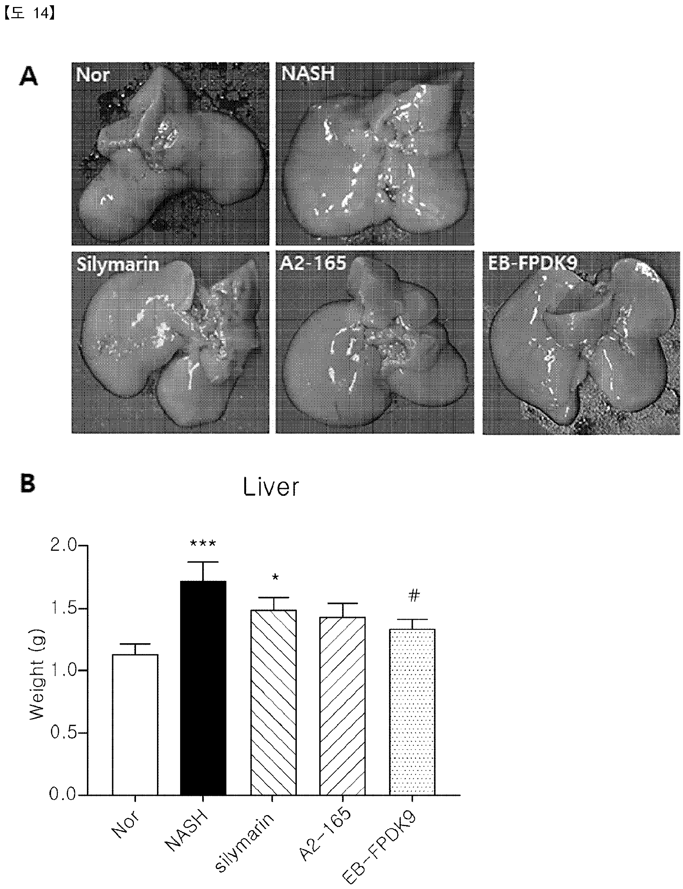

Referring to FIG. 14, it was observed that the liver tissue of the normal diet group showed a bright reddish healthy liver shape, whereas the liver of the group with nonalcoholic steatohepatitis induced by the experimental diet became cloudy in color due to lipid accumulation and lost the original bright reddish color. However, the silymarin-administered group, the *Faecalibacterium prausnitzii* A2-165 standard strain-administered group and the EB-FPDK9 strain-administered group showed a bright reddish liver shape close to that of the normal diet group. As a result of measuring the liver weight, the weight gain in each of the nonalcoholic steatohepatitis-induced group and the silymarin-administered group was observed compared to the normal diet group. However, the weight of the liver tissue of the *Faecalibacterium prausnitzii* EB-FPDK9 strain-administered group was most similar to that of the normal diet group, and did significantly differ from that of the nonalcoholic steatohepatitis-induced group. Through the results in FIG. 14, it was confirmed that the *Faecalibacterium prausnitzii* EB-FPDK9 strain-administered group exhibited a liver shape and weight similar to those of the normal diet group. Therefore, it could be concluded that the *Faecalibacterium prausnitzii* EB-FPDK9 strain can alleviate nonalcoholic steatohepatitis.

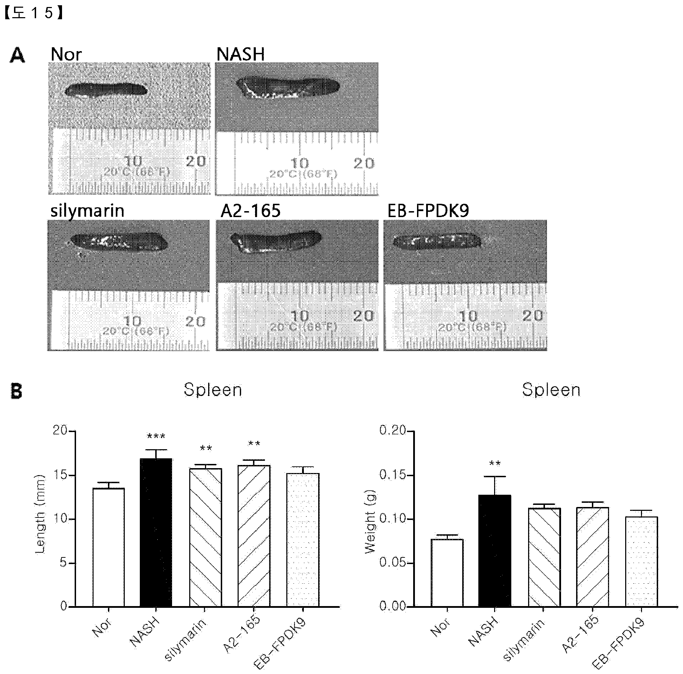

As shown in FIG. 15, the length and weight of the spleen increased in the nonalcoholic steatohepatitis-induced group compared to the normal diet group. Like the case of the liver tissue, it was observed that the length of the spleen of the group treated with each of silymarin and the *Faecalibacterium prausnitzii* A2-165 standard strain also increased compared to that of the normal diet group, but the increase in the spleen length in the *Faecalibacterium prausnitzii* EB-FPDK9 strain-administered group was so low that it was insignificant. It was confirmed that the weight of the spleen was lower than that of the non-alcoholic steatohepatitis-induced group.

### 5.5. Analysis of Blood Lipid Biochemical Indicators

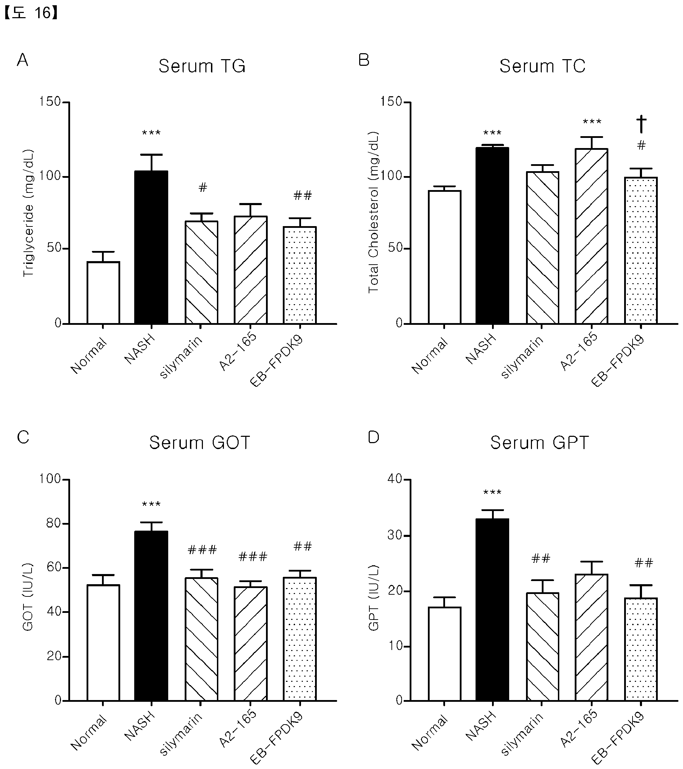

After fasting for 18 hours, blood was collected from each experimental animal, and then the concentrations of triglyceride (TG) and total cholesterol (TC), which are indicators of lipid content, and glutamic oxaloacetic transaminase (GOT) and glutamic pyruvic transaminase (GPT), which are indicators of liver function, in the serum isolated from the blood, were measured. The results of the measurement are shown in FIG. 16. The concentrations of TG, TC, GOT and GPT, which are lipid composition indicators, were all quantified using an individual measurement kit purchased from Asan Pharmaceutical Co., Ltd.

It was confirmed that the triglyceride concentration significantly increased in the nonalcoholic steatohepatitis-induced group. However, the triglyceride concentration significantly decreased in the silymarin-administered group and the *Faecalibacterium prausnitzii* EB-FPDK9 strain-administered group compared to the nonalcoholic steatohepatitis-induced group. The total cholesterol level was higher in the nonalcoholic steatohepatitis-induced group and the *Faecalibacterium prausnitzii* A2-165 standard strain-treated group compared to the normal group. However, the total cholesterol level significantly decreased in the *Faecalibacterium prausnitzii* EB-FPDK9 strain-treated group compared to the nonalcoholic steatohepatitis-induced group and the *Faecalibacterium prausnitzii* A2-165 standard strain-treated group. It was observed that the GOT concentration indicating the degree of hepatocellular damage decreased in all the administered groups compared to the nonalcoholic steatohepatitis-induced group, and that the GPT concentration significantly decreased only in the silymarin-administered group and the *Faecalibacterium prausnitzii* EB-FPDK9 strain-administered group. Through the analysis of blood lipid biochemical indicators, it was confirmed that the concentrations of triglycerides, total cholesterol, GOT, and GPT, which are closely related to nonalcoholic steatohepatitis, were decreased by administration of the *Faecalibacterium prausnitzii* EB-FPDK9 strain.

### 5.6. Analysis of Pathological Severity of Steatohepatitis in Liver Tissue

In order to observe the effect of administration of the *Faecalibacterium prausnitzii* EB-FPDK9 strain on the alleviation of nonalcoholic steatohepatitis, hematoxylin & eosin (H&E) staining of liver tissue sections, and Sirius red staining that can measure liver fibrosis, were performed, and the expression of alpha-smooth muscle actin (α-SMA), which occurs upon liver damage, was observed by staining. The liver tissue isolated from each mouse was sectioned to a thickness of about 5 um and then embedded in paraffin, and the difference in morphological changes was observed through each staining. The degree of liver damage observed through each staining was expressed as the percent positive area (%) through the Image J program.

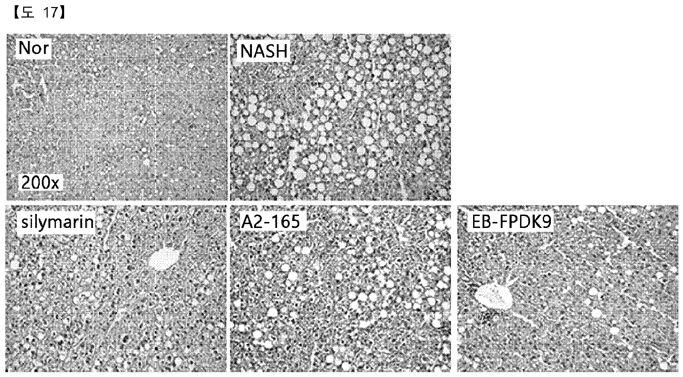

As shown in FIG. 17, as a result of analyzing the mouse liver tissue through H&E staining, it was observed that the liver tissue of the normal group had no fat droplet because the hepatocyte structure thereof was normally dense. However, in the liver tissue of the nonalcoholic steatohepatitis-induced mouse, the formation of a large number of fat droplets could be clearly observed compared to that in the normal group. It was observed that the formation of fat droplets decreased in all the administered groups compared to the nonalcoholic steatohepatitis-induced group, and it was confirmed that the formation of fat droplets further decreased in the silymarin-administered group and the *Faecalibacterium prausnitzii* EB-FPDK9 strain-administered group.

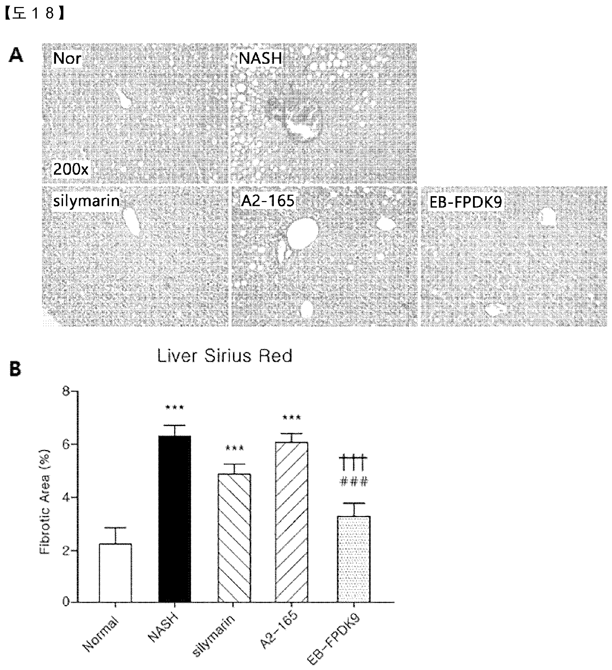

**As** shown in FIG. 18, the amount of collagen deposited was analyzed through Sirius red staining of the mouse liver tissue. The amount of collagen deposited in the liver is known as a sensitive indicator that reflects the degree of fibrosis. In this experiment, liver fibrosis increased in all the nonalcoholic steatohepatitis-induced group, the silymarin-administered group and the *Faecalibacterium prausnitzii* A2-165 standard strain-administered group compared to the normal group. However, it was confirmed that collagen production was significantly inhibited in the *Faecalibacterium prausnitzii* EB-FPDK9 strain-administered group compared to the nonalcoholic steatohepatitis-induced group and the *Faecalibacterium prausnitzii* A2-165 standard strain-administered group, suggesting that liver damage caused by liver fibrosis was significantly suppressed in the *Faecalibacterium prausnitzii* EB-FPDK9 strain-administered group.

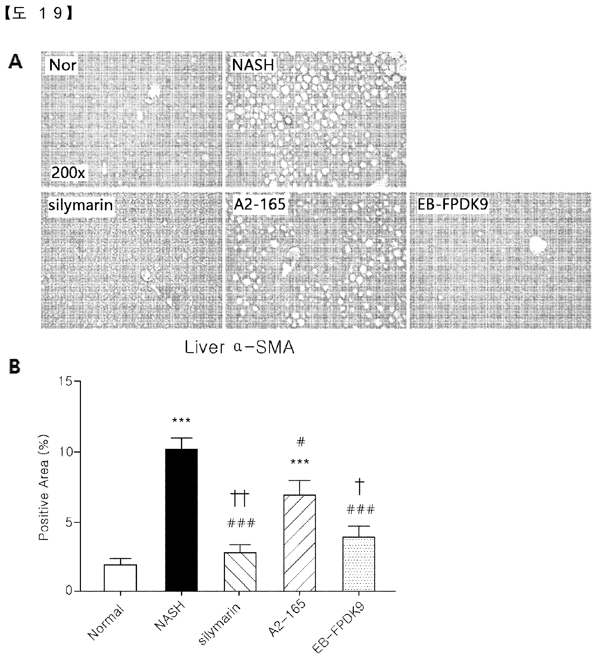

In addition, as a result of observing the degree of liver damage by observing the expression of α-SMA in mouse liver tissue through staining, as shown in FIG. 19, it was observed that the expression of α-SMA decreased in all the administered groups compared to the nonalcoholic steatohepatitis-induced group, suggesting that liver damage in these groups was suppressed. In addition, it was observed that the expression of α-SMA more significantly decreased in the silymarin-administered group and the *Faecalibacterium prausnitzii* EB-FPDK9 strain-administered group compared to the *Faecalibacterium prausnitzii* A2-165 standard strain-administered group.

### 5.7. Analysis of Triglycerides and Total Cholesterol Levels in Liver Tissue

Triglycerides as lipid extracts and total cholesterol in the mouse liver tissue were analyzed. 120 µl of PBS was added to 30 mg of the liver tissue which was then minced using a homogenizer, and then 320 µl of chloroform and 160 µl of MeOH were added thereto to obtain a mixture. The mixture was incubated in a shaking incubator at room temperature for one day, and then centrifuged at 2,000 rpm, and only the supernatant was separated and the solvent was evaporated therefrom. Thereafter, the supernatant from which the solvent has been evaporated was dissolved in 1 ml of isopropanol, and then quantified relative to the total liver weight of each mouse using a TG/TC measurement kit (Asan Pharmaceutical Co., Ltd., Korea).

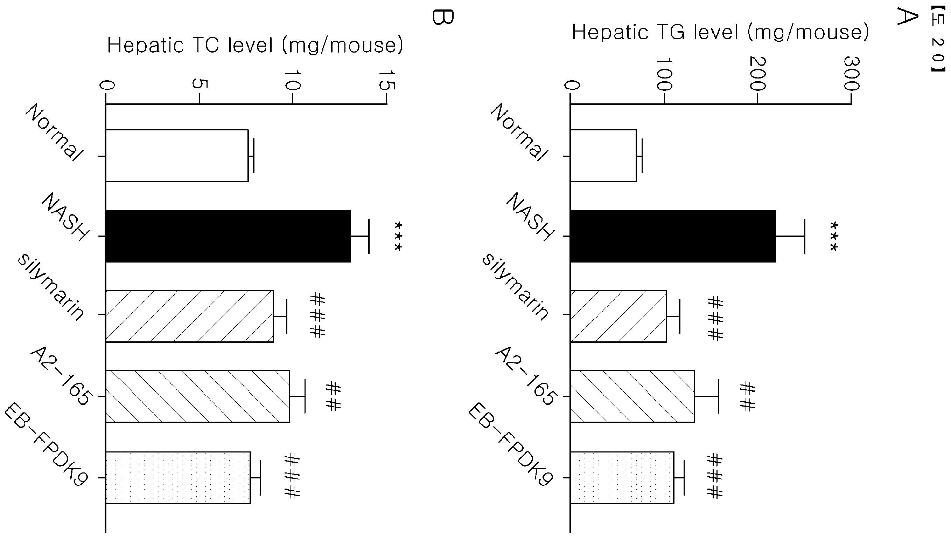

**As** a result, as shown in the graphs of FIG. 20, it was confirmed that the triglyceride level in the liver tissue significantly increased in the nonalcoholic steatohepatitis-induced group. However, in the silymarin-administered group, the *Faecalibacterium prausnitzii* A2-165 standard strain-administered group and the *Faecalibacterium prausnitzii* EB-FPDK9 strain-administered group, the triglyceride level significantly decreased. In addition, the total cholesterol level in the liver tissue was higher in the nonalcoholic steatohepatitis-induced group than in the normal group. However, the total cholesterol level in the liver tissue significantly decreased in the silymarin-administered group, the *Faecalibacterium prausnitzii* A2-165 standard strain-administered group and the *Faecalibacterium prausnitzii* EB-FPDK9 strain-administered group compared to the nonalcoholic steatohepatitis-induced group.

As a result of analyzing lipid accumulation in the liver tissue, it was confirmed that administration of the *Faecalibacterium prausnitzii* EB-FPDK9 strain along with silymarin most significantly inhibited the production of triglycerides and cholesterol and had the effect of ameliorating nonalcoholic steatohepatitis.

As a result of analyzing the liver tissue, it was confirmed that the progression of steatohepatitis and liver damage induced by nonalcoholic steatohepatitis was most significantly inhibited in the *Faecalibacterium prausnitzii* EB-FPDK9 strain-administered group among the administered groups.

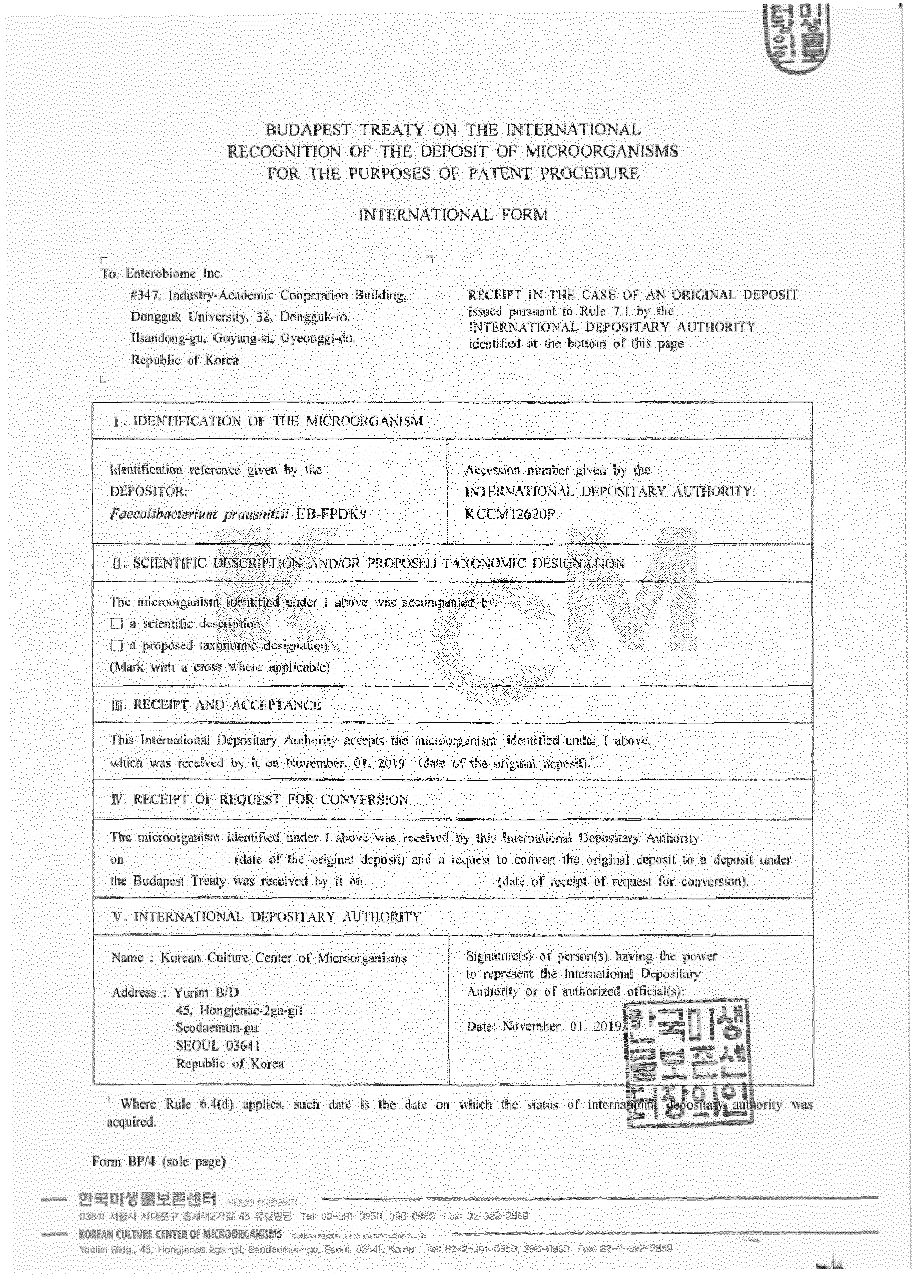
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Enterobiome Inc.
#347, Industry-Academic Cooperation Building,
Dongguk University, 32, Dongguk-ro,
Ilsandong-gu, Goyang-si, Gyeonggi-do,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Faecalibacterium prausnitzii EB-FPDK9 | Accession number given by the
INTERNATIONAL DEPOSITARY AUTHORITY:
KCCM12620P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on November. 01. 2019 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 01. 2019.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS [?]
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A *Faecalibacterium prausnitzii* EB-FPDK9 strain (accession number: KCCM12620P).

2. A pharmaceutical composition for preventing or treating inflammatory disease, the pharmaceutical composition containing at least one selected from the group consisting of the strain of claim 1, a culture of the strain, a lysate of the strain, and an extract of the strain.

3. A pharmaceutical composition for preventing or treating liver disease, the pharmaceutical composition containing at least one selected from the group consisting of the strain of claim 1, a culture of the strain, a lysate of the strain, and an extract of the strain.

4. A pharmaceutical composition for preventing or treating metabolic disease, the pharmaceutical composition containing at least one selected from the group consisting of the strain of claim 1, a culture of the strain, a lysate of the strain, and an extract of the strain.

5. A food composition for preventing or ameliorating inflammatory disease, the food composition containing at least one selected from the group consisting of the strain of claim 1, a culture of the strain, a lysate of the strain, and an extract of the strain.

6. A food composition for preventing or ameliorating liver disease, the food composition containing at least one selected from the group consisting of the strain of claim 1, a culture of the strain, a lysate of the strain, and an extract of the strain.

7. A food composition for preventing or ameliorating metabolic disease, the food composition containing at least one selected from the group consisting of the strain of claim 1, a culture of the strain, a lysate of the strain, and an extract of the strain.
